# EUROPÄISCHE PATENTSCHRIFT

(11) **EP 1 273 566 B1**
(45) Veröffentlichungstag und Bekanntmachung des Hinweises auf die Patenterteilung: **30.08.2006**
(21) Anmeldenummer: 02014219.6
(22) Anmeldetag: 26.06.2002
(51) Int. Cl.: C07C 67/03, C07C 69/24

(54) **Verfahren zum kontinuierlichen Herstellen von Esterölen**
Process for the continuous production of ester oils
Procédé de préparation en continu d'huiles d'esters

(30) Priorität: 05.07.2001 DE 10132677
(43) Veröffentlichungstag der Anmeldung: 08.01.2003
(73) Patentinhaber: Cognis IP Management GmbH, 40589 Düsseldorf (DE)
(72) Erfinder: Kubersky, Hans Peter, Dr., 42651 Solingen (DE); Schleper, Bernard, 40764 Langenfeld (DE); Wollmann, Gerhard, Dr., 40923 Hilden (DE)

(56) Entgegenhaltungen:
- EP-A- 0 469 607
- US-A- 5 767 257
- US-A- 5 945 529

## Beschreibung

Die Erfindung betrifft ein Verfahren zum kontinuierlichen Herstellen von Esterölen, die aus Estern aus einer aliphatischen C6-C10-Monocarbonsäure und einem höheren Alkohol bestehen, durch Umestern eines Methyl- oder Ethylesters der Monocarbonsäure mit dem höheren Alkohol unter homogener alkalischer Katalyse, wobei die Einsatzstoffe im gleichen Temperaturbereich sieden und die Siedetemperaturen sich insbesondere nur um höchstens 10 °C unterscheiden.

Esteröle sind seit längerem bekannt und werden unter anderem in großen Mengen als Bestandteil von Schmierstoffen eingesetzt. Diese Anmeldung bezieht sich insbesondere auf die Herstellung von Octansäure-2-Ethyl-Hexylester, die Erfindung ist aber nicht auf diesen Ester eingeschränkt, sondern das Herstellungsverfahren kann auch auf andere ähnliche Ester bzw. Einsatzstoffe mit ähnlichen Eigenschaften Verwendung finden.

Ganz allgemein sind ferner kontinuierliche Umesterungsverfahren bekannt, in denen man ein Gemisch eines Esters und eines Alkohols, der einen schwerer flüchtigen Ester liefert, sowie den Umesterungskatalysator dem oberen Teil einer Destillationskolonne zuführt und diese derart beheizt, dass der freiwerdende Alkohol über Kopf abdestilliert und der schwerer flüchtige Ester am Boden der Kolonne anfällt, wo er abgezogen werden kann.

Octansäure-2-Ethyl-Hexylester (CAS 63321-70-0), im folgenden als OEH abgekürzt, wird als Trägerflüssigkeit für petrofreie, ölbasierte Bohrspülungen und als Lampenöl eingesetzt.

Bekannt ist die Herstellung von Octansäure-2-Ethyl-Hexylester durch Batch-Synthese aus 2-Ethylhexanol (im folgenden als ETH bezeichnet) und kurzkettigem Methylester C6-10 sowie durch Batch-Synthese aus 2-Ethylhexanol und kurzkettiger Fettsäure. Als Reaktor wird ein Rührkesselreaktor mit Dephlegmator bzw. Kolonne und Vakuumsystem eingesetzt. Der Überschuss an 2-Ethylhexanol wird nach der Reaktion abdestilliert.

Bekannt ist es außerdem, zur kontinuierlichen Herstellung von Produkten aus mehreren Reaktanden eine Reaktionskolonne mit einer Anzahl von Glockenböden einzusetzen. Bei Reaktionskolonnen wird normalerweise ein Reaktand auf dem obersten Reaktionsboden als Flüssigkeit aufgegeben. Die Flüssigkeit strömt von oben nach unten über die Böden durch die Kolonne.

Damit sich die Flüssigkeit auf den Böden anstauen kann und das Reaktionsgemisch eine gewisse Verweilzeit in der Reaktionskolonne hat, ist auf einem Reaktionsboden das Ablaufwehr mit z. B. 150 mm Höhe höher als bei Rektifikationskolonnen mit Glockenböden.

Im kontinuierlichen Betrieb wird bei klassischen Reaktionskolonnen unten in die Kolonne ein zweiter Reaktand dampfförmig zugeführt. Er perlt durch die Flüssigkeit auf den Böden und reagiert mit dem flüssig zugeführten Reaktand.

Am Sumpf der Reaktionskolonne wird das Reaktions-Wertprodukt abgezogen. Am Kopf der Reaktionskolonne strömt der dampfförmige Überschuss des dampfförmig zugeführten Reaktanden und das dampfförmige Reaktionsnebenprodukt in den Kondensator und wird dort vollständig kondensiert. Um das Reaktionsnebenprodukt schnell aus dem Reaktionsgemisch zu entfernen, kann die Kolonne bzw. können die Böden beheizt werden.

Im kontinuierlichen Betrieb ist der gesamte flüssig zugeführte Reaktand am Sumpf der Kolonne nicht immer vollständig zum Reaktions-Wertprodukt umgesetzt. Der Reaktand kann in einem Sumpfverdampfer bei dem Druck der Reaktionskolonne nur teilweise aus dem Reaktionswertprodukt abdestilliert werden.

Beim Abfahren der Kolonne sollen ohne Gasbelastung die Böden leerlaufen können. Deshalb besitzt jede Glocke eines Drosselbohrung. Unter Normalbetrieb muss in einer Drosselbohrung mit 11 mm Durchmesser die Gasgeschwindigkeit größer als 8 m/s sein, damit die Böden nicht durchregnen.

Der Erfindung liegt die Aufgabe zugrunde, eine kontinuierliche Herstellung von Esterölen durch ein Verfahren der eingangs genannten Art zu ermöglichen, wobei schon nach einer geringen Verweilzeit ein praktisch vollständiger Umsatz erreicht wird. Weitere Vorteile der Erfindung finden sich nachfolgend.

Diese Aufgabe wird im Verfahren der eingangs genannten Art erfindungsgemäß dadurch gelöst, dass man das Verfahren in einer Reaktionskolonne mit einer Mehrzahl von Böden durchführt, welche ohne Gasbelastung leerlaufen, dass man die flüssigen Einsatzstoffe im oberen Teil der Kolonne zuführt und dass man ein Inertgas als Stützgas von unten nach oben durch die Reaktionskolonne strömen lässt.

Vorzugsweise stellt man Octansäure-2-Ethyl-Hexylester (OEH), insbesondere aus Octansäure-Methylester (ME) und 2-Ethylhexanol (ETH), her.

Als Inertgas setzt man vorzugsweise Stickstoff ein.

Insbesondere setzt man als Katalysator ein Alkoholat eines Alkalimetalls, insbesondere Natrium-Methylat, und ein trockenes, erwärmtes Inertgas ein, das die Einsatzstoffe trocknet. Die Trocknung mit dem Inertgasstrom verringert den Feuchtigkeitsgehalt der Einsatzstoffe, bevor der Katalysator mit der Feuchtigkeit reagieren kann. Der Katalysator Natrium-Methylat bleibt daher aktiv, denn es wird keine Natronlauge gebildet, und ohne Natronlauge im Reaktionssystem findet nur eine geringe Seifenbildung der Methylester statt. Ohne die Trocknung durch das Inertgas wäre der Katalysator Natrium-Methylat nicht oder nur unter großen Schwierigkeiten einsetzbar.

Vorzugsweise setzt man 30 %iges methanolisches Natrium-Methylat als Katalysator ein, welches im 2-Ethylhexanol gelöst sein kann.

Die Reaktionskolonne kann über ihre gesamte Länge beheizt sein. Möglich ist es aber auch, dass nur die oberen Böden beheizt werden. Daher wird vorgeschlagen, dass zumindest die oberen Böden der Reaktionskolonne beheizt sind.

Erfindungsgemäß strömen die Einsatzstoffe im Gleichstrom von oben nach unten durch die Reaktionskolonne.

Weiterhin wird vorgeschlagen, dass man den durch die Reaktionskolonne strömenden Inertgas-Strom (Gasbelastung) auf den minimalen Wert einstellt, bei welchem ein Durchregnen der Einsatzstoffe durch die Böden nicht auftritt.

Da im erfindungsgemäßen Verfahren am Sumpf der Kolonne praktisch der gesamte oben aufgegebene Ester, insbesondere Methylester, umgesetzt ist, arbeitet die Reaktionskolonne in einer weiteren Ausgestaltung der Erfindung ohne Sumpfverdampfer.

Weitere vorteilhafte Einzelmaßnahmen werden im folgenden für die Herstellung von Octansäure-2-Ethyl-Hexylester im einzelnen genannt.

### Zu den Einsatzstoffen

Einsatzstoffe für die Synthese von Octansäure-2-Ethyl-Hexylester (OEH) sind 2-Ethylhexanol (ETH) und Methylester (ME), vorzugsweise reiner Methylester C8 (ME C8). Bei Einsatz eines Methylester-C8-C10-Gemisches sollte der C6-Anteil so gering wie möglich sein, um die Verluste an Einsatz-Methylester zu minimieren.

ETH (T_{Siede} = 184 °C, p = 1 bar-a) und ME C8 (T_{Siede} = 192 °C, p = 1 bar-a) haben vergleichbare Siedebereiche. Deshalb ist die bekannte Fahrweise einer Reaktionskolonne mit der Zugabe einer Flüssigkeit am Kopf der Kolonne und einer dampfförmigen Zufuhr der anderen Komponente am unteren Boden nicht möglich.

Frisches ME C8 (158 kg/kmol) wird erfindungsgemäß auf dem obersten Reaktionsboden nur leicht angewärmt zugeführt. Die obersten Böden werden zum Trocknen des Methylesters genutzt und haben außerdem die Funktion eines Dephlegmators. Bei bekannten Reaktionskolonnen dagegen wird der flüssige Reaktand heiß zugeführt.

2-Ethylhexanol wird so trocken wie möglich zugeführt. Frisches ETH (130 kg/kmol) wird erfindungsgemäß als Flüssigkeit auf dem zweitobersten Reaktionsboden oder kurz darunter heiß zugeführt.

ETH und ME strömen erfindungsgemäß im Gleichstrom von oben nach unten durch die Kolonne.

### Zur Hydrodynamik und zur Stickstoffzufuhr

Damit die Böden hydrodynamisch stabil arbeiten und die Flüssigkeit nicht durch die Drosselbohrungen durchregnet, wurde als besonders geeignete Gasbelastung 7,8 m/s < w_{Luft} < 40 m/s ermittelt.

Als Stützgas für alle Böden mit ihren unterschiedlichen Drosselbohrungen kommt dampfförmiges ETH nicht in Frage, da es nach wenigen durchströmten Böden vollständig kondensieren würde. Die darüber liegenden Böden würden leerlaufen. Als Stützgas ist dampfförmiges Methanol ebenfalls nicht geeignet, da es sich im OEH lösen und im Reaktionsgleichgewicht einen hohen Umsatz zu OEH verhindern würde.

Als Stützgas wird erfindungsgemäß Stickstoff eingesetzt. Der Stickstoff wird vorzugsweise über einen Erhitzer erhitzt. Es wird vorzugsweise eine solche Menge an Stickstoff auf dem untersten Boden zugeführt, dass die Böden auch bei vollständig abgelaufener Reaktion nicht durchregnen. Die vergleichbare, auf Luft bezogene Strömungsgeschwindigkeit w_{Luft} des Stickstoffs in den Drosselbohrungen ist vorzugsweise über 7,8 m/s eingestellt, wie es bereits oben angegeben worden ist.

Der Gasleerrohrgeschwindigkeit bezogen auf den Kolonnenquerschnitt ist vorzugsweise gleich oder größer als 0,06 m/s und insbesondere kleiner als 0,079 m/s.

### Zum Katalysator

Als Katalysator wird vorzugsweise 30 %ige methanolische Na-Methylat-Lösung (Na-M) eingesetzt.

Die Na-Methylat-Lösung wird in die Kolonne separat auf Höhe der ETH-Zufuhr zugeführt. Alternativ und bevorzugt kann die Na-Methylat-Lösung auch zusammen mit dem ETH zugeführt werden.

Vorzugsweise wird 1,1 bis 3 mol Na-Methylat/kmol frischer Methylester zudosiert.

Bei hoher Restfeuchtigkeit im Reaktionsgemisch könnte das Na-Methylat mit Wasser zu NaOH reagieren. Das NaOH würde dann die Methylester verseifen. Die Seifen würden auf den Reaktionsböden ausfallen, die Glocken verstopfen und der Druckverlust würde ansteigen.

Daher sollte die Na-Methylat-Lösung keinen direkten Kontakt mit dem feuchten Methylester bzw. ME-haltigem Recycle-Produkt bekommen, da sonst die Feuchtigkeit im Methylester dessen Verseifung bewirkt. Beim Vorliegen von NaOH bzw. KOH im Reaktionsgemisch springt die gewünschte Reaktion außerdem nicht oder nur sehr langsam an.

Obwohl ETH 0,1 % Wasser enthält und im ME C8-10 noch 0,24 % Wasser gemessen werden, erfolgt erfindungsgemäß kein separater Trocknungsschritt der Rohstoffe. Bei den bekannten Batch-Prozessen werden die Rohstoffe dagegen etwa 1 h lang getrocknet.

Vorzugsweise dient das Stützgas Stickstoff auch zum Trocknen der Einsatzstoffe. Der Stickstoffstrom senkt beim Durchströmen der obersten Böden den Partialdruck und die Feuchtigkeit wird aus den Einsatzstoffen abgetrieben, bevor das Wasser den Katalysator Na-Methylat desaktivieren kann.

### Zum ETH-Überschuss

Vorzugsweise wird molar nur soviel frisches ETH zugeführt, wie ME zugeführt wird. Vorzugsweise liegt in der Kolonne ein ETH-Überschuss von 1,3 bis 1,6 mol ETH/mol ME vor. Vorzugsweise wird der Überschuss an ETH im Kreis gefahren. Der erste Kreislauf besteht vorzugsweise aus: Kopf der Reaktionskolonne, Rektifikationsaufsatz, Partialkondensator, warmer Rücklauf.

Vom Kopf der Reaktionskolonne steigen die N2-MeOH-ETH-ME-Brüden auf und werden partiell in einem Dephlegmator kondensiert. Das ETH-ME-Kondensat strömt als Rücklauf auf den Fraktionierteil oberhalb der Reaktionskolonne zurück. Die Temperatur des Dephlegmators wird vorteilhaft so eingestellt, dass der Rücklauf 5 bis 15 °C wärmer als die Kondensationstemperatur von Methanol ist. Nur wenig Methanol kondensiert daher, welches als Brüden aus dem Dephlegmator zusammen mit dem gasförmigen Hauptanteil des Methanols und mit dem Stickstoffstrom ausgetragen wird. Bei tieferen Kondensationstemperaturen würde mehr Methanol kondensieren und im oberen Teil der Kolonne im Kreis gefahren werden. Die Umsatzrate würde sinken.

Vorzugsweise wird ein zweiter Kreislauf genutzt, welcher besteht aus: Sumpf der Reaktionskolonne, Abtriebskolonne, Brüden kondensieren, Kondensat warm auf obersten Reaktionsboden zurückführen.

Im Sumpf der Reaktionskolonne ist noch nicht umgesetzter Methylester und der ETH-Überschuss enthalten. In einer Abtriebskolonne wird der Rest-ME und der ETH-Überschuss aus dem OEH-roh abgetrennt. Erfindungsgemäß wird das Überschuss-Destillat in die Reaktionskolonne zurückgeführt. Vorzugsweise wird das Überschuss-Destillat zusammen mit frischem ME dem obersten Reaktionsboden warm zugeführt. Das Ziel ist ein hoher ETH-Überschuss in den oberen Reaktionszonen. Dann ist dort mit einer hohen Reaktionsrate zu rechnen und die Hauptmenge an Methanol kann auf den obersten, beheizten Böden direkt verdampfen. Alternativ kann der recyclierte ETH-Überschuss direkt mit dem frischen ETH zugeführt werden.

### Zur Kühlfalle und zum Wäscher

In der Kühlfalle, die dem Dephlegmator nachgeschaltet ist, wird mitgeschleppter ETH und ME zusammen mit Wasser und dem Reaktionsnebenprodukt Methanol soweit wie möglich aus dem Stickstoffstrom kondensiert. Bei 10 °C Kondensationstemperatur in der Kühlfalle werden 95 % des Methanols kondensiert. Der verbleibende Gasstrom nach der Kühlfalle enthält neben Methanol und Stickstoff nur noch Spuren von ETH und ME.

Im nachgeschalteten Wäscher wird mit wenig Wasser das Methanol aus dem Gasstrom ausgewaschen.

### Zur Filtration und Destillation des rohen Octansäure-2-Ethyl-Hexylesters (OEH)

Das OEH-roh nach der Abtriebskolonne enthält noch Katalysator und Seifenanteile. Er ist gefärbt.

Die Seifenanteile lassen sich durch Filtration aus dem kalten OEH abfiltrieren. Das Filtrat ist klar.

Vorteilhaft ist es, den OEH-roh nicht kalt werden zu lassen, damit die Seifen gelöst bleiben und das OEH problemlos über Kopf destilliert werden kann. Bei einem Einsatz von 1,1 mol Na-Methylat/kmol ME fallen je nach Destillationsbedingungen 2 bis 6 % Sumpf an. Das OEH-Destillat ist wasserklar. Es enthält nur Spuren an ETH (weniger als 0,3 %). Vorteilhaft ist als Destillationstemperatur die Siedetemperatur des OEH zu wählen, z. B. beim Einsatz von reinem OEH ex ME C8 einen Druck von 15 mbar und eine Temperatur von etwa 148 °C.

Zusammengefasst wird das erfindungsgemäße Verfahren zur kontinuierlichen Herstellung von Octansäure-2-Ethyl-Hexylester in einer Reaktionskolonne mit Destillation des Überschusses an 2-Ethylhexanol folgendermaßen durchgeführt:

Weil Methylester C8 (ME) und 2-Ethylhexanol (ETH) im gleichen Siedebereich sieden, strömen sie im Gleichstrom durch die Reaktionskolonne von oben nach unten. Als Katalysator dient 30 %iges methanolisches Na-Methylat, das im ETH gelöst sein kann. Als Stützgas, damit die Böden nicht durchregnen, strömt erwärmtes Inertgas, insbesondere Stickstoff von unten nach oben durch die beheizte Kolonne. Gleichzeitig trocknet der Stickstoffstrom die Einsatzstoffe. Ein gesonderter Trocknungsschritt ist nicht erforderlich. Damit bleibt der Katalysator Na-Methylat aktiv und wird nicht mit der Restfeuchte der Einsatzstoffe zu NatronLauge umgesetzt. Ohne Natronlauge im System kommt es nicht zur Seifenbildung der Methylester.

Besonders auf den oberen Böden läuft die Reaktion zu Octansäure-2-Ethyl-Hexylester ab. Das Reaktionsprodukt Methanol verdampft und verlässt schnell das Reaktionsgemisch. Das Reaktionsgleichgewicht verschiebt sich schnell zu Octansäure-2-Ethyl-Hexylester. Mit etwa 45 Minuten Verweilzeit (bezogen auf den Feed) ist am Sumpf der Kolonne praktisch der gesamte Methylester umgesetzt. Als ETH-Überschuss reichen 1,3 mol ETH/mol ME aus.

In einer Abtriebskolonne lässt sich der ETH-Überschuss aus dem Reaktionsprodukt unter Vakuum abtrennen. Die Sumpftemperatur der Abtriebskolonne ist die Siedetemperatur des OEH. Emulgierte Seifenanteile lassen sich aus dem kalten OEH-roh abfiltrieren.

Zur Abtrennung des Katalysators und der Farben kann der Octansäure-2-Ethyl-Hexylester OEH noch über Kopf destilliert werden. Das OEH-Destillat ist wasserklar. Die Restmengen an ETH im Destillat sind kleiner als 0,3 %.

### Anlage zur Durchführung des Verfahrens

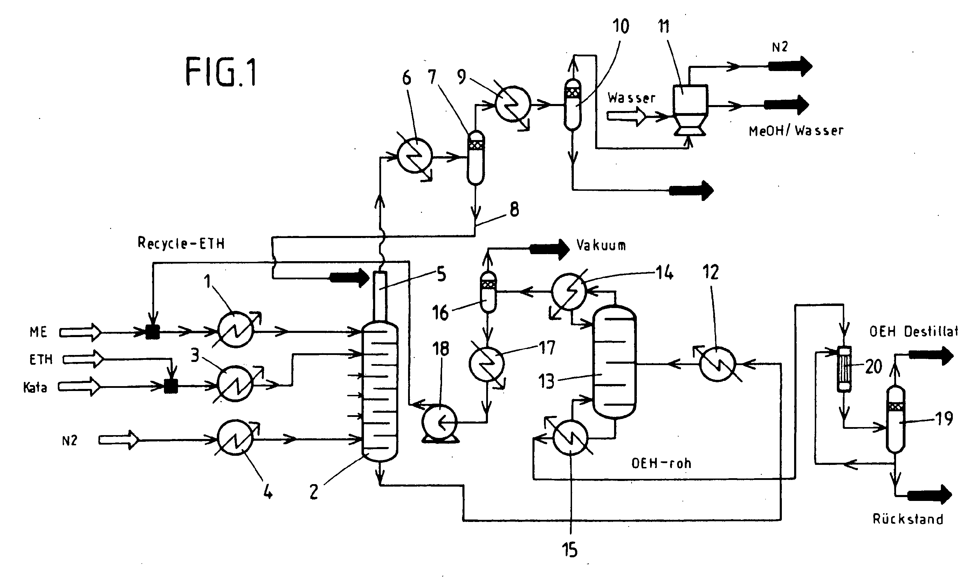

In Figur 1 ist ein Fließbild einer Anlage zum Durchführen des erfindungsgemäßen Verfahrens beispielhaft dargestellt.

Der frische Methylester ME wird zusammen mit den rückgeführten ETH in einem Wärmetauscher 1 leicht angewärmt und dem obersten Boden einer Reaktionskolonne 2 zugeführt. Frisches ETH wird zusammen mit dem Katalysator in einem weiteren Wärmetauscher 3 erhitzt und ebenfalls dem oberen Teil der Reaktionskolonne 2, aber einem Boden unterhalb des obersten Bodens zugeführt. Über einen Erhitzer 4 wird Stickstoffgas dem untersten Boden der Reaktionskolonne 2 zugeführt.

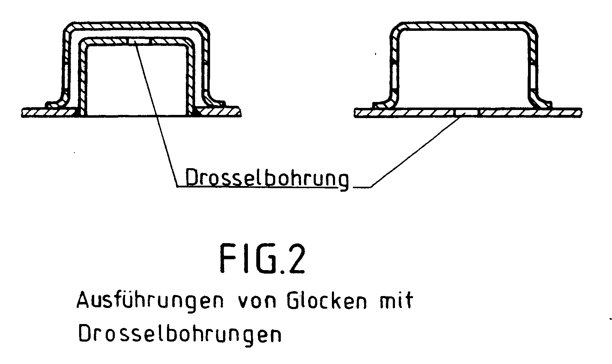

An sich bekannte Glockenböden der Rektifikationskolonne 2 sind in Figur 2 im Längsschnitt durch die Rektifikationskolonne dargestellt. Der Überschuss an ETH wird in zwei Kreisläufen rückgeführt. Vom Rektifikationsaufsatz 5 der Reaktionskolonne 2 steigen Brüden auf, die aus Stickstoffgas, Methanol, ETH und ME bestehen. Sie werden nach einer Kühlung im Wärmetauscher 6 partiell in einem Dephlegmator 7 kondensiert. Das aus ETH und ME bestehenden Kondensat strömt über die Rücklaufleitung 8 zum Rektifikationsaufsatz 5 der Reaktionskolonne 2 zurück. Die Brüden, die aus dem Dephlegmator 7 aufsteigen, bestehen im wesentlichen aus Methanol und Stickstoff. Sie werden nach einer weiteren Abkühlung im Wärmetauscher 9 zur Kühlfalle 10 geleitet, wo ETH, ME und Wasser sowie das Reaktionsnebenprodukt Methanol kondensieren. Der verbleibende Gasstrom enthält nur noch wenig Methanol sowie nur noch Spuren von ETH und ME sowie den gesamten Stickstoffanteil. Dieser Gasstrom wird im nachgeschalteten Wäscher 11 mit wenig Wasser behandelt, wobei das Methanol ausgewaschen wird und als Gasanteil schließlich nur noch Stickstoff verbleibt.

Das Sumpfprodukt der Rektifikationskolonne 2 wird über einen weiteren Erhitzer 12 einem mittleren Boden einer Abtriebskolonne 13, welche unter Vakuum betrieben wird, zugeführt. Die Abtriebskolonne 13 ist mit einem Kühler 14 am Kopf sowie mit einem Sumpferhitzer 15 ausgestattet. Über eine Kühlfalle 16 und einen weiteren Kühler 17 wird das am Kopf der Abtriebskolonne 13 erhaltene Produkt als Kondensat über eine Pumpe 18 warm dem obersten Reaktionsboden der Rektifikationskolonne 2 zugeführt.

Das Sumpfprodukt der Abtriebskolonne 13, welches das gewünschte Rohprodukt OEH-roh darstellt, wird in einer Destillationskolonne 19, welche mit einem Fallfilmverdampfer 20 ausgestattet ist, über Kopf destilliert. Das Destillat stellt das gewünschte Endprodukt OEH dar.

### Erfindungsgemäßes Beispiel:

Einer Reaktionskolonne 2 mit 17 Böden und mit einem Hold up von 0,15 I/Boden wurde 1,3 kg/h ME C8 mit einer Temperatur von 75 °C zugeführt. Auf dem dritten Boden von oben wurden 1,07 kg/h ETH zusammen mit 0,43 kg/h ETH-Überschuss mit einer Temperatur von 160 °C zugeführt. Im ETH waren 1,2 g 30 %iges methanolisches Na-Methylat/kg ETH gelöst. Auf dem untersten Boden wurde 1,2 m³/h Stickstoff (Leerrohrgeschwindigkeit 0,059 m/s) mit einer Temperatur von 160 °C zugeführt.

Die Reaktionskolonne 2 wurde auf 160 °C beheizt. Ein vorhandener Sumpfverdampfer wurde nicht beheizt, so dass die Kolonne 2 ohne Sumpfverdampfer arbeitete. Der Rücklauf aus dem Dephlegmator 6,7 hatte eine Temperatur von 70 °C. Das Kondensat der Kühlfalle wurde nicht zurückgeführt. In einem wassergefüllten Wäscher 11 wurde das Rest-Methanol aus dem Stickstoff-Brüdenstrom ausgewaschen.

Die Hauptreaktion lief besonders auf den obersten Böden schnell ab, und die Hauptmenge an Methanol verdampfte dort sofort. Es stellte sich entlang der Längsrichtung der Kolonne 2 ein Umsatzprofil ein. Der ETH-Überschuss wurde entlang der Kolonne aus dem Reaktionsprodukt ausgetrieben. Die Zusammensetzung in der Reaktionskolonne 2, gemessen in Flächen-% mit einem Gaschromatographen, ist in der folgenden Tabelle 1 in Abhängigkeit von der Verweilzeit VWZ in Minuten im Reaktionsraum dargestellt.

**Tabelle 1:**

| VWZ | ETH | ME | OEH |
|---|---|---|---|
| 8 | 75,7 | 5,2 | 19,1 |
| 19 | 67,3 | 1,1 | 31,6 |
| 31 | 66,2 | 0,4 | 33,4 |
| 35 | 52,2 | 0,2 | 47,6 |
| 41 | 23,9 | 0,06 | 76,0 |

Bei allen Versuchen, bei denen die Temperatur nicht zu hoch war, wurde im Waschwasser des Wäschers 2 keine obere Phase beobachtet. Das Waschwasser enthielt 17 % Methanol und ca. 0,1 % ETH.

Bei allen Versuchen ohne Rückführung von Kondensat der Kühlfalle 10 war die Seifenbildung auf den Reaktionsböden gering. Die Seifen waren im warmen Zustand gelöst und fielen nicht aus. Der Druckverlust in der Reaktionskolonne 2 entsprach dem hydrostatischen Druckverlust.

In einer Abtriebskolonne 13 mit Fallfilmverdampfer 15 wurde der ETH-Überschuss abdestilliert. Der Feed wurde mit einer Temperatur von 120 °C in die Mitte der Kolonne zugeführt. Bei 100 mbar und einem Rücklaufverhältnis von R/D = 1:4 stellten sich die folgenden Temperaturen ein:

| | |
|---|---|
| am Kopf | 115 °C |
| am Feed-Boden | 121 °C |
| unterste Zone | 175 °C |
| im Sumpf | 204 °C |

Am Sumpf der Abtriebskolonne fiel das OEH-roh an. Das OEH-roh enthielt weniger als 0,3 % ETH. Das Produkt OEH-roh war hellgelb und zeigte im warmen Zustand keine Seifenschlieren. Jedoch war es trübe.

Aus dem trüben, erkalteten OEH-roh ließen sich Seifenanteile abfiltrieren. Das filtrierte OEH-roh war hellgelb und klar. Es konnte direkt als Bohrspülmittel eingesetzt werden.

Um Farben und den Katalysator vollständig abzutrennen, wurde aus einem unfiltrierten OEH-roh das OEH bei 15 mbar und 148 °C Verdampfer-Temperatur in der Kolonne 19 destilliert. Es fiel 6 % Sumpf an. Das Destillat war wasserklar und enthielt weniger als 0,3 % ETH.

### Bezugszeichenliste

| | |
|---|---|
| 1 | Wärmetauscher |
| 2 | Reaktionskolonne |
| 3 | Wärmetauscher |
| 4 | Erhitzer |
| 5 | Rektifikationsaufsatz |
| 6 | Wärmetauscher |
| 7 | Dephlegmator |
| 8 | Rücklaufleitung |
| 9 | Wärmetauscher |
| 10 | Kühlfalle |
| 11 | Wäscher |
| 12 | Erhitzer, Fallfilmverdampfer |
| 13 | Abtriebskolonne |
| 14 | Kühler |
| 15 | Sumpferhitzer |
| 16 | Kühlfalle |
| 17 | Kühler |
| 18 | Pumpe |
| 19 | Destillationskolonne |
| 20 | Fallfilmverdampfer |

## Patentansprüche

1. Verfahren zum kontinuierlichen Herstellen von Esterölen, die aus Estern aus einer aliphatischen C6-C10-Monocarbonsäure und einem höheren Alkohol bestehen, durch Umestern eines Methyl- oder Ethylesters der Monocarbonsäure mit dem höheren Alkohol unter homogener alkalischer Katalyse, wobei die Einsatzstoffe im gleichen Temperaturbereich sieden und die Siedetemperaturen sich insbesondere nur um höchstens 10 °C unterscheiden,
**dadurch gekennzeichnet,**
**dass** man das Verfahren in einer Reaktionskolonne (2) mit einer Mehrzahl von Böden durchführt, welche ohne Gasbelastung leerlaufen, dass man die flüssigen Einsatzstoffe im oberen Teil der Kolonne (2) zuführt und dass man ein Inertgas als Stützgas von unten nach oben durch die Reaktionskolonne (2) strömen lässt.

2. Verfahren nach Anspruch 1,
**dadurch gekennzeichnet,**
**dass** man Octansäure-2-Ethyl-Hexylester (OEH), insbesondere aus Octansäure-Methylester (ME) und 2-Ethylhexanol (ETH), herstellt.

3. Verfahren nach einem der vorhergehenden Ansprüche,
**dadurch gekennzeichnet,**
**dass** man als Inertgas Stickstoff einsetzt.

4. Verfahren nach einem der vorhergehenden Ansprüche,
**dadurch gekennzeichnet,**
**dass** man als Katalysator ein Alkoholat eines Alkalimetalls, insbesondere Natrium-Methylat, und ein trockenes, erwärmtes Inertgas einsetzt, das die Einsatzstoffe trocknet.

5. Verfahren nach dem vorhergehenden Anspruch,
**dadurch gekennzeichnet,**
**dass** man 30 %iges methanolisches Natrium-Methylat als Katalysator einsetzt.

6. Verfahren nach einem der vorhergehenden Ansprüche,
**dadurch gekennzeichnet,**
**dass** zumindest die oberen Böden der Reaktionskolonne (2) beheizt sind.

7. Verfahren nach einem der vorhergehenden Ansprüche,
**dadurch gekennzeichnet,**
**dass** die Einsatzstoffe im Gleichstrom von oben nach unten durch die Reaktionskolonne (2) strömen.

8. Verfahren nach einem der vorhergehenden Ansprüche,
**dadurch gekennzeichnet,**
**dass** man den durch die Reaktionskolonne (2) strömenden Inertgas-Strom (Gasbelastung) auf den minimalen Wert einstellt, bei welchem ein Durchregnen der Einsatzstoffe durch die Böden nicht auftritt.

9. Verfahren nach einem der vorhergehenden Ansprüche,
**dadurch gekennzeichnet,**
**dass** die Reaktionskolonne (2) ohne Sumpfverdampfer arbeitet.

## Claims

1. Process for the continuous production of ester oils consisting of esters of an aliphatic C₆₋₁₀ monocarboxylic acid and a higher alcohol by transesterification of a methyl or ethyl ester of the monocarboxylic acid with the higher alcohol in the presence of a homogeneous alkaline catalyst, the starting materials boiling in the same temperature range and their boiling temperatures differing in particular by only at most 10°C, **characterized in that** the process is carried out in a reaction column (2) with a plurality of trays which drain without a gas load, **in that** the liquid starting materials are delivered to the upper part of the column (2) and **in that** an inert gas as support gas is allowed to flow upwards through the reaction column (2).

2. Process as claimed in claim 1, **characterized in that** octanoic acid-2-ethylhexyl ester (OEH) is produced, more particularly from octanoic acid methyl ester (ME) and 2-ethylhexanol (ETH).

3. Process as claimed in any of the preceding claims, **characterized in that** nitrogen is used as the inert gas.

4. Process as claimed in any of the preceding claims, **characterized in that** an alcoholate of an alkali metal, more particularly sodium methylate, is used as the catalyst and a dry heated inert gas that dries the starting materials is used.

5. Process as claimed in the preceding claim, **characterized in that** 30% methanolic sodium methylate is used as the catalyst.

6. Process as claimed in any of the preceding claims, **characterized in that** at least the upper trays of the reaction column (2) are heated.

7. Process as claimed in any of the preceding claims, **characterized in that** the starting materials flow downwards through the reaction column (2) in co-current.

8. Process as claimed in any of the preceding claims, **characterized in that** the inert gas stream (gas load) flowing through the reaction column (2) is adjusted to the minimum value at which the starting materials do not drop through the trays.

9. Process as claimed in any of the preceding claims, **characterized in that** the reaction column (2) operates without a bottom evaporator.

## Revendications

1. Procédé de préparation en continu d'huiles esters constituées d'esters d'un acide monocarboxylique aliphatique en C6 à C10 et d'un alcool supérieur, par transestérification d'un ester méthylique ou éthylique de l'acide monocarboxylique avec l'alcool supérieur sous catalyse alcaline homogène, les substances mises en oeuvre entrant en ébullition dans la même plage de température et les températures d'ébullition ne se distinguant en particulier que de 10°C au plus,
**caractérisé en ce qu'**
on met en oeuvre le procédé dans une colonne de réaction (2) comportant une multitude de plateaux qui fonctionnent à vide sans charge de gaz, on induit les substances liquides mises en oeuvre à la partie supérieure de la colonne (2) et, en tant que gaz support, on fait passer un gaz inerte de bas en haut à travers la colonne de réaction (2).

2. Procédé selon la revendication 1,
**caractérisé en ce qu'**
on prépare de l'ester 2-éthyl-hexylique de l'acide octanoïque (OEH), en particulier à partir d'ester méthylique (ME) de l'acide octanoïque et de 2-éthylhexanol (ETH).

3. Procédé selon l'une des revendications précédentes,
**caractérisé en ce qu'**
on utilise de l'azote comme gaz inerte.

4. Procédé selon l'une quelconque des revendications précédentes,
**caractérisé en ce que**
comme catalyseur, on utilise un alcoolate d'un métal alcalin, en particulier, du méthylate de sodium, et un gaz inerte réchauffé sec qui sèche les substances mises en oeuvre.

5. Procédé selon l'une des revendications précédentes,
**caractérisé en ce que**
comme catalyseur, on utilise du méthylate de sodium méthanolique à 30%.

6. Procédé selon l'une des revendications précédentes,
**caractérisé en ce qu'**
on chauffe au moins les plateaux supérieurs de la colonne de réaction (2).

7. Procédé selon l'une des revendications précédentes,
**caractérisé en ce que**
les substances mises en oeuvre s'écoulent dans le même sens de haut en bas à travers la colonne de réaction (2).

8. Procédé selon l'une des revendications précédentes,
**caractérisé en ce qu'**
on ajuste le courant de gaz inerte traversant la colonne de réaction (2) (charge de gaz) à la valeur minimale permettant d'éviter un arrosage des substances mises en oeuvre à travers les plateaux.

9. Procédé selon l'une des revendications précédentes,
**caractérisé en ce que**
la colonne de réaction (2) fonctionne sans évaporateur de fond de colonne.
